# EUROPEAN PATENT APPLICATION

(11) **EP 1 728 854 A1**
(43) Date of publication of application: **06.12.2006**
(21) Application number: 05104845.2
(22) Date of filing: 03.06.2005
(51) Int. Cl.: C12N 1/16, C12N 1/18, A21D 8/04, A23L 1/03

(54) **Process for the production of yeast biomass**

(71) Applicant: DSM IP Assets B.V., 6411 TE Heerlen (NL)
(72) Inventor: de Winde, Johannes Hendrik, 2215 WC Voorhout (NL)
(74) Representative: Misset, Onno

(57) **Abstract**

The present invention describes a process for the production of yeast biomass on a commercial scale by culturing yeast cells under aerobic conditions in a growth medium which comprises at least one assimilable carbon source and recovering the yeast cells to obtain yeast biomass, characterised in that the yeast cells functionally overexpress a transcriptional activator such, that the yeast cells have an increased respiratory capacity and an increased fermentative capacity in comparison with the yeast cells not overexpressing the transcriptional activator. The invention also describes the use of yeast biomass according to the invention in the preparation of a food product.

## Description

The present invention relates to a process for the production of yeast biomass on a commercial scale by culturing yeast cells under aerobic conditions in a growth medium which comprises at least one assimable carbon source, and recovering the yeast cells to obtain yeast biomass, yeast functionally overexpressing a transcriptional activator and to the use of yeast according to the invention in the preparation of a food product.

To date, yeast is used in a large variety of different biotechnological processes. *Saccharomyces cerevisiae,* also known as baker's, wine or brewer's yeast is probably the most well known representative, but other yeasts, albeit more or less closely related to this yeast have recently become or are currently becoming of biotechnological importance as well. The purpose and utility of the various biotechnological processes applying yeast can be broadly divided into two major categories, depending on the physiology and metabolic state of the yeast cells obtained in and during the process: 1) fermentative growth and 2) respiratory growth.

Under fermentative growth conditions, yeast converts carbon sources into mainly ethanol and CO₂. Examples of fermentative processes are the production of ethanol in beverages such as wine and beer, or use as a solvent or a fuel, and for the production of CO₂ in beer and for the leavening of dough in the production of bread.

Under respiratory, or aerobic growth conditions, yeast converts carbon sources completely into oxidised end products, while consuming oxygen (O₂). These respiratory conditions are normally applied during production of yeast biomass, for instance in batch, fed-batch or continuous cultures. The yeast biomass thus obtained can subsequently be used in the fermentative processes described above, or can be converted to yeast extracts to be used as savoury flavours in the food industry. Alternatively, the production of yeast biomass may be carried out for the concomitant production of (homologous or heterologous) enzymes, proteins (eg. pharmaceutical proteins), nutrients or other biotechnologically relevant metabolites.

The yeast is very well able to adapt its cellular composition, metabolism and physiological state towards each mode of growth, i.e. respiratory of fermentative growth. These modes differ considerably in the yield of biomass on substrate. For the production of yeast biomass, it is important that the respiratory capacity is high, whereas for the application of yeast in a fermentative process it is important that the fermentative capacity of the yeast is sufficiently high. Both modes of growth need to be dealt with satisfactorily to arrive at optimised production. For example, both types of growth are necessary for the baking industry. One challenge in the baker's yeast production is to optimise the efficiency of conversion of assimable carbon into biomass during the production phase, while ensuring that the yeast will have good leavening characteristics in the dough. Other respiratory yeast cultivation processes have similar complicated demands for balance between biomass production and optimal end-use characteristics (flavour, heterologous protein production, etc.).

Several yeasts, such as *Saccharomyces cerevisiae,* show a preference for certain carbon sources, in particular glucose, which is used above all other sources of energy. The reason for this preference is that high glucose concentrations repress a number of metabolic functions, including the respiratory capacity, through a process known as glucose repression (Gancedo, 1998, Microbiol. Mol. Biol. Rev 62: p. 334-361). Glucose-repressed cells start accumulating ethanol, which reduces the yield of yeast biomass in an aerobic, or respiratory production process.

Various studies that have been carried out to improve the yield of yeast biomass production, were directed on increasing the respiratory capacity or the carbon metabolism of yeast. WO 00/61722 describes a yeast wherein genes involved in glucose repression of the yeast (*HXK2* genes or analogues thereof) have been deleted. Consequently, the yeast comprising the *HXK2* gene deletion was able to grow to a higher yield in a respiratory process than the parent yeast, while the fermentative capacity of the yeast remained unaffected. Alternatively, WO 98/26079 describes a yeast in which the glucose repression is reduced or absent, by controlled de-regulation of the glucose-repressible genes by overexpressing a specific transcriptional activator, HAP4 or analogues thereof, from a promotor insensitive to glucose. The yeast thus obtained exhibited an increased respiratory capacity resulting in an increased yield of biomass, but the fermentative capacity of the yeast remained unaffected as in WO 00/61722.

Despite these studies, there remains a strong desire to not only increase the yield of yeast biomass during aerobic production (i.e. increase the respiratory capacity) but also to, at the same time, improve the application properties of the yeast under anaerobic conditions (i.e. increase the fermentative capacity).

The object of the present invention is to provide an alternative process for the production of yeast biomass, resulting in a yeast with an increased respiratory capacity and concomitantly an increased fermentative capacity in comparison with a yeast known in the prior art.

The respiratory capacity may be defined as consumption rate of O₂ per unit biomass.

The fermentative capacity may be expressed as the production rate of carbon dioxide and/or ethanol formed per unit of biomass.

Yeast biomass may be defined as accumulated yeast or accumulated yeast cells.

As defined herein, the yield is the amount of yeast biomass per amount of substrate.

The productivity of the fermentor may be defined as the amount of yeast biomass, per volume and per unit of time.

In one aspect the present invention provides a process for the production of yeast biomass on a commercial scale by culturing yeast cells under aerobic conditions in a growth medium which comprises at least one assimilable carbon source and recovering the yeast cells to obtain yeast biomass, whereby the yeast cells functionally overexpress a transcriptional activator such, that the yeast cells have an increased respiratory capacity and an increased fermentative capacity in comparison with the yeast cells not overexpressing said transcriptional activator.

Yeast cells having an increased respiratory capacity according to the invention is understood to encompass yeast cells having an at least 2%, preferably at least 5%, preferably at least 10%, preferably at least 20%, more preferably at least 30%, most preferably at least 50% higher O₂ consumption rate compared with the yeast cells not overexpressing the transcriptional activator and grown under identical conditions.

Yeast cells having an increased fermentative capacity according to the invention is understood to encompass an at least 2%, preferably at least 5%, preferably at least 10%, preferably at least 20%, more preferably at least 30%, most preferably at least 50% higher carbon dioxide and/or ethanol production rate compared with the yeast cells not overexpressing the transcriptional activator and fermented under identical conditions.

Any suitable transcriptional activator may be functionally overexpressed in yeast cells as long as the functionally overexpressed activator leads to yeast cells with an increased respiratory capacity and concomitantly an increased fermentative capacity compared to yeast cells not overexpressing the transcriptional activator. Preferably, the transcriptional activator is Hap1 or a functional equivalent or a homologue thereof. Hap1 (*H*eme activated protein 1) is a transcriptional activator that is known to regulate a number of genes involved in respiratory metabolism in yeast in response to the availability of oxygen (O₂). More detailed information on the Hap1 transcription activator may be found in for example Kwast et al., 1998, J. Exp. Biol. 201: 1177-1195. The Hap1 transcriptional activator may be found in several microorganisms such as the yeasts *Saccharomyces cerevisiae* and *Kluyveromyces lactis* (Ha et al. 1996 Nucl. Acids Res. 24: 1453-1459), and the fungus *Aspergillus nidulans* (Johnson and Bradshaw, Fungal Genetics Newsletter # 45).

Homologues of Hap1 are understood to encompass proteins with an amino acid sequence which is at least 40%, preferably at least 50%, more preferably at least 60%, more preferably at least 70%, and most preferably at least 80% homologous to the amino acid sequence of the Hap1 amino acid sequence of *Saccharomyces cerevisiae* (Creusot et al. 1988, J. Mol. Biol. 204, 263-276 ; Pfeifer et al. 1989, Cell 56, 291-301).

A functional equivalent of Hap1 is understood to encompass proteins which have at least the same functional properties as the Hap1 of *Saccharomyces cerevisiae,* namely that of transcriptional activator.

In the scope of the present invention, functional overexpression of the transcripional activator implies that the activity of the transcriptional activator is increased. Functional overexpression of the transcriptional activator may be obtained in any suitable way, for example by increasing the copy number of a gene encoding the transcriptional activator, which results in an increased intracellular concentration of said transcriptional activator. Functional overexpression of the transcriptional activator may also be obtained by introduction in the protein dominant amino acid mutations that increase DNA binding affinity and transcriptional activation by the activator. The mutations may be effected either through spontaneous (i.e. classical) mutagenesis procedures and strain selection, or through recombinant DNA approaches. All these methods are known methods for the skilled person in the art.

The transcriptional activator may be expressed constitutively, or upon induction, for instance by the presence of oxygen. In many instances it will be preferred that the vector used to introduce the activator is capable of integration into the genome of the host. In another preferred embodiment, a self-replicating vector may be used.

Any suitable promotor may be used by the yeast cells to overexpress the transcriptional activator, for instance any constitutive promoter. Preferably, said promoter comprises the *HAP1* promoter region.

Any yeast may be used in the process according to the invention. Suitable strains are for example those belonging to the genus *Saccharomyces, Hansenula, Torulaspora* or *Kluyveromyces.* Preferably, the yeast in the process according to the invention is *Saccharomyces cerevisiae, Torulaspora delbrueckii, Kluyveromyces lactis.*

Culturing yeast cells under aerobic conditions may be performed in several ways, which are all known in the art: batch, fed-batch, or continuous culture, a combination of batch and fed-batch mode, repeated fed-batch mode or any other combination. Batch mode fermentation processes are characterised by the fact that the growth medium containing the substrates is added to the fermenter followed by inoculation of the medium with a preculture of the microorganism. Fed-batch fermentation processes are characterised by the fact that the complete medium containing the substrates is not added at the onset of the fermentation process, but part of the medium is added as a continuous or intermittent feed. For instance, glucose or other essential nutrients are fed to the culture in a growth limiting fashion. Production processes under continuous culture conditions resemble the fed-batch processes except that biomass-containing effluent is removed from the fermentation continuously or intermittently. All these ways of culturing the yeast are known methods for the skilled person in the art.

In a continuous (or chemostat) culture, the yeast is cultured at a steady state dilution rate, which equals the growth rate (µ). Preferably, the steady state dilution rate is between 0.02 to 0.35 h⁻¹, more preferably between 0.05 and 0.30 h⁻¹, most preferably between 0.08 and 0.25 h⁻¹.

Culturing yeast cells on a commercial scale is understood to encompass culturing yeast cells in a volume of at least 0.5 litre, preferably = 5 litres, preferably = 10 litres, preferably = 20 litres, more preferably = 50 litres, more preferably = 100 litres, more preferably = 1 m³, even more preferably = 10 m³, most preferably = 100 m³. The actual commercial scale depends on the amount of yeast biomass required for a given application.

The growth medium that is used in the process according to the invention may be any medium which is known in the art, suitable for growing yeast cells. The growth medium comprises at least one assimilable carbon source for example a carbohydrate. Examples of suitable carbohydrates are glucose, fructose, maltose, saccharose, galactose and raffinose. Preferably, the assimilable carbon source used in the process according to the invention is molasses.

Yeast cells comprising the functionally overexpressed transcriptional activator, and which have been cultured under aerobic conditions, may be recovered and accumulated from the culture medium to produce yeast biomass in any suitable way. The yeast biomass may for example be recovered by centrifugation or filtration.

In a second aspect, the invention provides yeast biomass obtainable by the process according to the invention. The yeast biomass obtainable by the process according to the invention may be obtained in an amount of at least 0.1 kg, preferably at least 0.5 kg, more preferably at least 1 kg, more preferably at least 10 kg, even more preferably at least 100 kg, most preferably at least 1000 kg, expressed as yeast dry matter. Determination of yeast dry matter can be carried out by known methods in the art.

In another aspect, the invention provides baker's yeast in the form of compressed yeast, cream yeast or dried yeast prepared from the yeast biomass according to the invention. The manufacture of baker's yeast on a commercial scale usually starts with a small sample of a pure culture. This sample is used to inoculate the first of a series of fermenters of successively increasing size. The first few fermentation stages are mildly aerated batch fermentations. In these stages, the conditions are such that ethanol is formed. Only the last two (or sometimes three) fermentation stages are performed using full aeration with incremental feeding of molasses. These last two fermentations may be fed-batch or continuous fermentations which are carried out in fermenters of 100 m³ or more net volume. The fermentation time is typically in the range of 12-20 hours, in which some 20,000-30,000 kg of fresh yeast is produced. After the feeding of substrates has stopped, aeration is usually continued at a reduced level for half an hour or so to let the yeast cells attain maturity and uniformity. Further processing includes separation of the yeast from the broth by centrifugation, washing and optionally diluting which results in cream yeast (10-24wt% dry matter content). The cream yeast thus obtained may be further processed to produce compressed yeast, cream yeast or dried yeast according to methods known in the art.

In a further aspect, the invention provides the use of yeast biomass according to the invention in the preparation of a food product.

The food product which is prepared with the yeast biomass according to the invention may be a beverage such as wine and beer. Preferably, the food product is a dough or the baked product thereof. A dough according to the invention comprises flour, water, and the yeast biomass obtainable by the process according to the invention. The preparation of dough involves a series of steps such as mixing, moulding and fermentation resulting in leavening of the dough and is known in the art. The dough thus obtained may subsequently be baked to prepare a baked product thereof. Examples of baked products according to the invention are a bread or a pizza.

### Description of the figures

**Figure 1:** Schematic representation of plasmid YEpHAP1, for overexpression of full length *HAP1* gene, including 1000 bp of upstream non-coding sequence and 300 bp of non-coding sequence downstream of the stop codon of the *HAP1* gene.
**Figure 2**: Schematic representation of the various steps of the fusion PCR procedure applied to construct a fusion gene between the *PMA1* promoter and the *HAP1* transcriptional activator gene.
**Figure 3:** Schematic depiction of plasmid YEpPMA-HAP1, for high constitutive overexpression of *HAP1* through the *PMA1* promoter.

The following examples are for illustrative purposes, and are not to be construed as being limitative to the invention.

### Examples

### Example 1

### Construction of a yeast overexpressing the HAP1 gene

Overexpression of the *HAP1* gene in baker's yeast *Saccharomyces cerevisiae* was achieved in the following ways.

### 1.1 Construction and application of a plasmid for overexpression of full length HAP1.

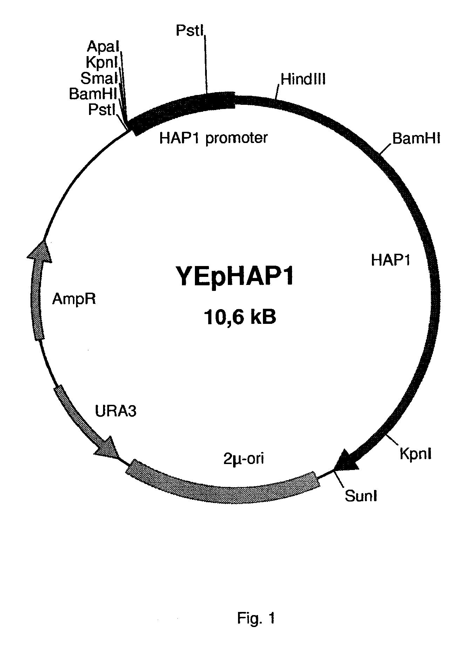

A full-length *HAP1* gene, including 900 bp of upstream promoter sequence and 250 bp of sequences following the translational stop codon was amplified and recovered from *Saccharomyces cerevisiae* haploid strain CEN.PK113-7D (*MAT**a**, MAL2-8C, SUC2)* and from aneuploid industrial strain DS28911 by PCR-based gene amplification. For this, the PCR primers HAPFW (SEQ ID No.1) and HAPRV (SEQ ID No. 2) were used in a polymerase chain reaction with yeast genomic DNA as template, deoxynucleotides and commercial DNA polymerase to produce a homogeneous mixture of *HAP1* containing DNA fragments. SEQ ID No.1 and SEQ ID No. 2 were designed using the *HAP 1* DNA sequences as published in Creusot et al. 1988, J. Mol. Biol. 204, 263-276; Pfeifer et al. 1989, Cell 56, 291-301 ; Johnston et al. (1997) Nature 387 - Suppl 6632, 87-90 and Gaisne et al. (1999) Curr. Genet. 36, 195-200. The *HAP1* fragments were cloned into a yeast -specific episomal plasmid Yeplac195, resulting in vector YEpHAP1 harbouring the full length *HAP1* gene, the *URA3* gene for positive marker selection in *ura3* auxotrophic yeast strains, and an *ARS1* sequence for autonomous DNA replication of the whole plasmid (**Figure 1**). The presence of a full length unaltered copy of the *HAP1* gene was verified by restriction fragment analysis and DNA sequence analysis. Cells of uracil-auxotrophic haploid yeast CEN.PK113-5D 7D (*MAT**a**, MAL2-8C, SUC2, ura3*) and of aneuploid industrial yeast DS28911-[*ura3*]₄ were transformed with YEpHAP1 and transformants were selected for uracil prototrophy. Overexpression of the *HAP1* gene in the transformants was verified by Northern analysis of total RNA with a *HAP1*-specific probe fragment.

### 1.2 Construction and application of a plasmid for high constitutive overexpression of HAP1.

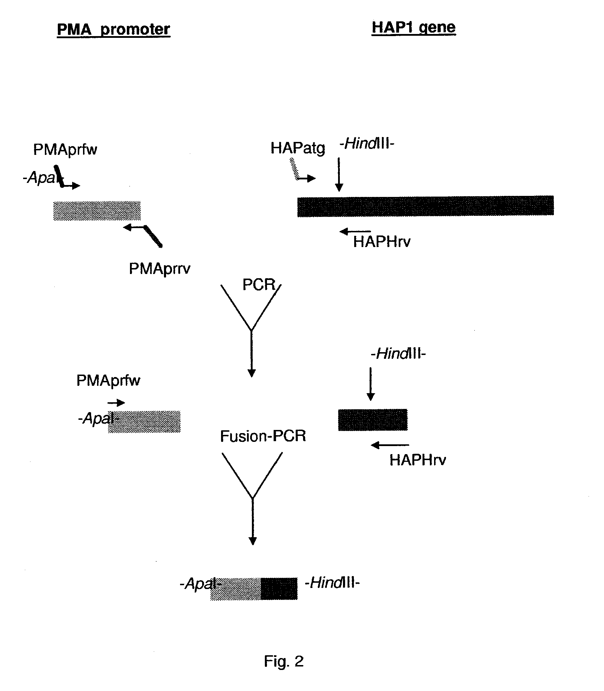

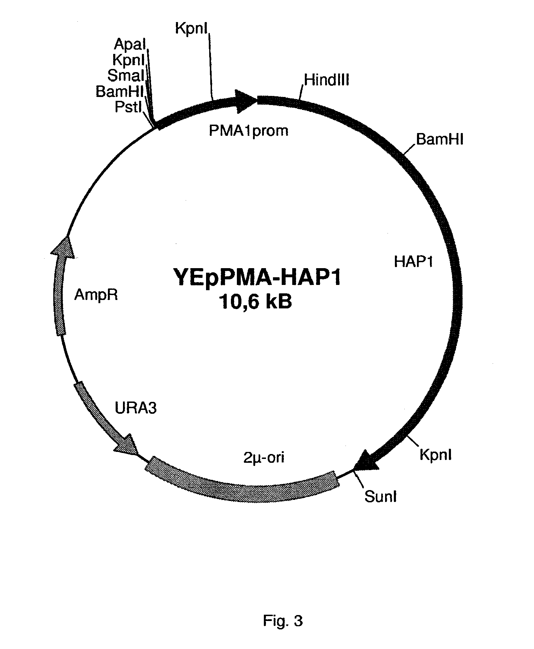

A fragment of the *HAP1* gene comprising the first 480 bp of the coding region from the ATG was amplified and recovered from *Saccharomyces cerevisiae* haploid strain CEN.PK113-7D (*MATa, MAL2-8C, SUC2*) and from aneuploid industrial strain DS28911 by PCR-based gene amplification. For this, the PCR primers HAPatg (SEQ ID No. 3) and HAPHrv (SEQ ID No. 4) were used in a polymerase chain reaction with yeast genomic DNA as template, deoxynucleotides and commercial DNA polymerase to produce a homogeneous mixture of DNA fragments containing the partial *HAP1* sequence (**Figure 2**). SEQ ID No. 3 and SEQ ID No. 4 were designed using the *HAP 1* DNA sequences as published in Creusot et al. 1988, J. Mol. Biol. 204, 263-276; Pfeifer et al. 1989, Cell 56, 291-301 ; Johnston et al. (1997) Nature 387 - Suppl 6632, 87-90 and Gaisne et al. (1999) Curr. Genet. 36, 195-200. In a parallel PCR-based DNA amplification reaction, a homogeneous mixture of DNA fragments containing the promoter region of the *PMA1* gene, encoding the yeast cytosolic membrane proton ATPase (Serrano et al. 1986, Nature 319, 689-693) was obtained, using PCR primers PMAprfw (SEQ ID No. 5) and PMAprrv (SEQ ID No. 6), total yeast genomic DNA, deoxynucleotides and commercial DNA polymerase (**Figure 2**). SEQ ID No. 5 and SEQ ID No. 6 were designed using the DNA sequence as published in Serrano et al. 1986, Nature 319, 689-693. The 5'-overhanging end of primer HAPatg harbours sequence homology to the extreme 3'-end of the PCR-amplified *PMA1*-promoter region, whereas the 5'-overhanging end of primer PMAprrv harbours sequence homology to the extreme 5'-end of the PCR-amplified *HAP1* fragment. Consequently, following PCR amplification the extreme 3'-end of the *PMA1*-promoter region and the 5'-end of the *HAP1* coding fragment harbour identical DNA sequence (**Figure 2**). Subsequently, the *PMA1* promoter fragments and the partial *HAP1* fragments were together used as DNA template in a PCR-based gene amplification reaction using PCR primers PMAprfw (SEQ ID No. 5) and HAPHrv (SEQ ID No. 4) deoxynucleotides and commercial DNA polymerase to produce fusion fragments containing the *PMA1*-promoter in front of the partial *HAP1* coding fragment (**Figure 2**). The fusion fragments were digested with Apal and HindIII restriction endonucleases and used to replace a Apal - HindIII fragment comprising the *HAP1* promoter and partial *HAP1* coding sequence from YEpHAP1, yielding YEpPMA-HAP1 containing a full length *HAP1* gene under the control of the *PMA1* promoter (**Figure 3**)**.** Proper coupling of the *PMA1* promoter with the *HAP1* coding region, as well as the presence of a full length unaltered copy of the *HAP1* gene in YEpPMA-HAP1 was verified by DNA sequence analysis. Cells of uracil-auxotrophic haploid yeast CEN.PK113-5D 7D (*MATa, MAL2-8C, SUC2, ura3*) and of aneuploid industrial yeast DS28911-[*ura3*]₄ were transformed with YEpPMA-HAP1 and transformants were selected for uracil prototrophy. Overexpression of the *HAP1* gene in the transformants was verified by Northern analysis of total RNA with a *HAP1*-specific probe fragment. A higher intensity of the corresponding band in the Northern blot showed that more mRNA was expressed than in Example 1.from the *HAP1* promoter.

### Example 2

### Production of yeast biomass whereby the yeast cells functionally overexpress the transcriptional activator Hap 1.

Cells of aneuploid industrial baker's yeast strain DS28911-[*ura3*]₄ transformed with Yeplac195 to relieve uracil auxotrophy, or transformed with YEpHAP1 were cultivated in continuous culture in carefully defined synthetic medium (Verduyn et al., 1992, Yeast 8, 501-517), at a fixed dilution rate of 0.1 h⁻¹. The oxygen uptake rate (OUR) is as given in **Table 1.**
The OUR is expressed as mmole oxygen (O₂) per kilogram yeast broth or yeast suspension, per hour (mmol/kg/h). The OUR was determined at the end of the batch-phase in the chemostat, before starting the continuous feed phase. OUR was also determined at steady state of growth of the continuous culture.

**Table 1.**

| | **Overexpression of HAP1** | **End of batch fase** | **Increase** | **Steady state 0.10 h**^{**-1**} | **Increase** |
|---|---|---|---|---|---|
| **Yeast** | | **OUR (mmol/kg/h)** | | **OUR (mmol/kg/h)** | |
| DS28911-[*ura3*]₄ | no | 25 | 0% | 23 | 0% |
| YEpHAP1 | yes | 41 | +64% | 37 | +61% |

**Fermentative capacity of yeast cells functionally overexpressing the transcriptional activator Hap 1.**
Yeast biomass was harvested from a continuous culture of strain DS28911-[*ura3*]₄ as described above transformed with Yeplac195, or transformed with YEpHAP1 grown to steady state at a fixed dilution rate of 0.1 h⁻¹. Biomass was washed and centrifuged to obtain yeast suspensions of comparable concentrations. This biomass was subsequently suspended in a defined liquid medium containing excess sugar, but otherwise sufficient amounts of various nutrients to sustain viability and metabolic activity while allowing only limited growth. In this medium the actual fermentative capacity of the yeast biomass was assessed as the production rate of CO₂ in mmole per gram dry weight of yeast, per hour (mmol/g/h). Results are depicted in **Table 2**.

**Table 2.**

| **Yeast** | **Overexpression of HAP1** | **Dilution rate** | **Fermentative capacity** (mmol/g/h) | **Increase** |
|---|---|---|---|---|
| DS28911 *ura3+* | no | 0.10 h⁻¹ | 13.3 | 0% |
| DS28911 pHAP1 | yes | 0.10 h⁻¹ | 18.9 | +42 % |

The results show that yeast overexpressing the transcriptional activator Hap1 exhibits an increased respiratory capacity and an increased fermentative capacity.

## Claims

1. Process for the production of yeast biomass on a commercial scale by culturing yeast cells under aerobic conditions in a growth medium which comprises at least one assimilable carbon source and recovering the yeast cells to obtain yeast biomass, **characterised in that** the yeast cells functionally overexpress a transcriptional activator such, that the yeast cells have an increased respiratory capacity and an increased fermentative capacity in comparison with the yeast cells not overexpressing the transcriptional activator.

2. Process according to claim 1, **characterised in that** the transcriptional activator is Hap1 or a functional equivalent or a homologue thereof.

3. Process according to any one of the claims 1 or 2 **characterised in that** the yeast cells functionally overexpress the transcriptional activator by using a constitutive promotor.

4. Process according to any one of the claims 1 to 3, **characterised in that** the yeast cells functionally overexpress the transcriptional activator by using a *HAP1* promotor region.

5. Process according to any one of the claims 1 to 4, **characterised in that** overexpressing the transcriptional activator is performed by increasing the copy number of the gene encoding the transcriptional activator.

6. Process according to any one of the claims 1 to 5, **characterised in that** the yeast belongs to the genus *Saccharomyces, Kluyveromyces, Hansenula* or *Torulaspora,* preferably the yeast is *Saccharomyces cerevisiae, Torulaspora delbrueckii, Kluyveromyces lactis.*

7. Process according to any one of the claims 1 to 6, **characterised in that** said culturing is performed in a continuous culture at a growth rate (µ) of between 0.02 to 0.35 h⁻¹.

8. Yeast biomass obtainable by the process according to any one of the claims 1 to 7.

9. Yeast biomass according to claim 8, in an amount of at least 0.1 kg.

10. Compressed yeast, cream yeast or dried yeast prepared from the yeast biomass according to claim 8 or 9.

11. Use of a yeast biomass according to claim 8 or 9 in the preparation of a food product.

12. Use according to 11, **characterised in that** the food product is a dough and a baked product thereof.

13. A dough comprising flour, water and yeast biomass according to claim 8 to 10.


### Annex to the application documents - subsequently filed sequences listing

SEQUENCE LISTING

<110> DSM IP Assets B.V.

<120> Process for the production of yeast biomass

<130> 21365EP/P2/

<160> 6

<170> PatentIn version 3.1

<210> 1
<211> 21
<212> DNA
<213> Artificial sequence
<220>
<221>
<223> Primer SEQ ID No. 1

<400> 1
ggcaagacgc gatcgtggcg g 21

<210> 2
<211> 22
<212> DNA
<213> Artificial sequence
<220>
<221>
<223> Primer SEQ ID No. 2

<400> 2
cccattctga aacgcaacgg ac 22

<210> 3
<211> 48
<212> DNA
<213> Artificial sequence
<220>
<221>
<223> Primer SEQ ID No. 3

<400> 3
gatttaatta tcaaacaata tcaatatgtc gaatacccct tataattc 48

<210> 4
<211> 21
<212> DNA
<213> Artificial sequence
<220>
<221>
<223> Primer SEQ ID No. 4

<400>  4
gtggtgtgtt catcgctacc c                                                21

<210>  5
<211>  42
<212>  DNA
<213>  Artificial sequence
<220>
<221>
<223>  Primer SEQ ID No. 5

<400>  5
atagcttggg cccagcgctt ccggtaattt gaaaacaaac cc                         42

<210>  6
<211>  52
<212>  DNA
<213>  Artificial sequence
<220>
<221>
<223>  Primer SEQ ID No. 6

<400>  6
gaattataag gggtattcga catattgata ttgtttgata attaaatctt tc              52

SEQUENCE LISTING

<110> DSM IP Assets B.V.

<120> Process for the production of yeast biomass

<130> 21365EP/P2/

<160> 6

<170> PatentIn version 3.1

<210> 1
<211> 21
<212> DNA
<213> Primer SEQ ID No. 1

<400> 1
ggcaagacgc gatcgtggcg g
21

<210> 2
<211> 22
<212> DNA
<213> Primer SEQ ID No. 2

<400> 2
cccattctga aacgcaacgg ac
22

<210> 3
<211> 48
<212> DNA
<213> Primer SEQ ID No. 3

<400> 3
gatttaatta tcaaacaata tcaatatgtc gaatacccct tataattc
48

<210> 4
<211> 21
<212> DNA
<213> Primer SEQ ID No. 4

<400> 4
gtggtgtgtt catcgctacc c
21

<210>  5
<211>  42
<212>  DNA
<213>  Primer SEQ ID No. 5

<400>  5
atagcttggg cccagcgctt ccggtaattt gaaaacaaac cc
    42


<210>  6
<211>  52
<212>  DNA
<213>  Primer SEQ ID No. 6

<400>  6
gaattataag gggtattcga catattgata ttgtttgata attaaatctt tc
    52